# EUROPEAN PATENT SPECIFICATION

(11) **EP 1 267 924 B1**
(45) Date of publication and mention of the grant of the patent: **08.02.2012**
(21) Application number: 01929778.7
(22) Date of filing: 26.03.2001
(51) Int. Cl.: A61K 39/39, A61K 9/127, C07K 14/785, A61P 31/00, A61P 33/00, A61P 35/00, A61P 37/00

(54) **IMMUNOTHERAPEUTIC METHODS AND COMPOSITIONS**
IMMUNTHERAPEUTISCHE METHODEN UND ZUSAMMENSETZUNGEN
METHODES ET COMPOSITIONS IMMUNOTHERAPEUTIQUES

(30) Priority: 24.03.2000 GB 0007150
(43) Date of publication of application: 02.01.2003
(73) Proprietor: Lamellar Biomedical Limited, Glasgow G2 5AP (GB)
(72) Inventor: DOBBIE, James, Airdrie ML6 9DZ (GB)
(74) Representative: Rutherford, Claire
(86) International application number: PCT/GB2001/001936
(87) International publication number: WO 2001/072277

(56) References cited:
- EP-A- 0 976 403
- WO-A-90/07469
- WO-A-92/21981
- WO-A-97/03703
- WO-A-98/50527
- MYERS K R ET AL: "THE IMMUNOLOGICAL ADJUVANT ACTIVITY OF LIPOSOMES CONTAINING MONOPHOSPHORYL LIPID A AND A SYNTHETIC ANALOG OF TREHALOSE DIMYCOLATE" FASEB (FEDERATION OF AMERICAN SOCIETIES FOR EXPERIMENTAL BIOLOGY), vol. 3, no. 3, 1989, page A605 XP002182355 1989 ISSN: 0892-6638
- GREGORIADIS G: "IMMUNOLOGICAL ADJUVANTS: A ROLE FOR LIPOSOMES" IMMUNOLOGY TODAY, ELSEVIER PUBLICATIONS, CAMBRIDGE, GB, vol. 11, no. 3, 1 March 1990 (1990-03-01), pages 89-97, XP000102991 ISSN: 0167-5699

### SEQUENCE LISTING

<110> Lamellar Therapeutics Ltd
<120> Immunotherapeutic methods and compositions
<130> Adjuvant peptides
<140>
   <141>
<160> 4
<170> PatentIn Ver. 2.1
<210> SEQ ID NO: 1
   <211> 12
   <212> PRT
   <213> Mycobacterium leprae
<400> 1
   Arg Gly Ile Glu Lys Ala Val Glu Lys Val Thr Glu
   1 5 10
<210> SEQ ID NO: 2
   <211> 12
   <212> PRT
   <213> Homo sapiens
<400> 2
   Arg Gly Lys Glu Gln Cys Val Glu Met Tyr Thr Asp
   1 5 10
<210> SEQ ID NO: 3
   <211> 16
   <212> PRT
   <213> Mycobacterium leprae
<400> 3
   Val Glu Glu Ser Asn Thr Phe Gly Leu Gln Leu Glu Leu Thr Glu Gly
   1 5 10 15
<210> SEQ ID NO: 4
   <211> 15
   <212> PRT
   <213> Homo sapiens
<400> 4
   Val Lys Lys Tyr Asn Thr Tyr Ala Tyr Val Gly Leu Thr Glu Gly
   1 5 10 15

## Description

The present invention relates in general to the field of immunotherapy, being therapies which act on or via the immune system. In particular, the invention relates to methods and compositions for introducing chemical entities into antigen presenting cells, particularly dendritic cells, resulting in the presentation of said antigens on the surface of the antigen presenting cells and a resulting effect on the immune system. The invention relates also to the resulting modified antigen presenting cells and pharmaceutical compositions containing these cells. Furthermore, the invention discloses new understandings in adjuvancy and adjuvant preparations including a series of related peptides and phospholipid vesicles incorporating said peptides.

At the present time there is considerable interest in the manipulation of the mammalian immune system by incorporating antigens into the surface of antigen presenting cells in general and dendritic cells in particular. By incorporating antigens into the surface of antigen presenting cells, the immune system can be made to target selected antigens. This has been applied to many therapeutic fields including cancer therapy. Many cancer antigens have been published and are being used in trials at the present time for human dendritic cell-based immunotherapy for cancer e.g. as disclosed in US 5,990,294 and 5,874,290 to Northwest Biotherapeutics, LLC.

It is, however, difficult to prepare dendritic cells with modified antigens on their surface. A number of techniques have been considered in the prior art. Currently known methods include "pulsing" antigenic peptide directly onto the surface of antigen presenting cells or incubating antigen presenting cells with whole proteins or protein fragments which are then ingested by the antigen presenting cells. These peptides are digested into small fragments by the antigen presenting cells and presented on their cell surfaces. These techniques are all of limited efficiency and reproducibility and relate only to peptide antigens.

Other methods have been published. For example, WO96/30030 to Baxter International Inc and WO98/46785 to Dana-Farber Cancer Institute disclose methods of fusing a patient's own dendritic cells with non-dendritic cells, e.g. cancer cells that express a cell-surface antigen to which it is desired to obtain an immune response. Such cells can then be grown and injected into a host, near their lymphoid system, leading to an immune response to the desired antigen. However, this process is complex and slow, requiring genetic engineering of a patient's cell lines in order to include a gene coding for the antigen of interest. Furthermore, they do not provide a method of displaying antigens which are not proteins as processed by the protein processing machinery of the resulting dendritic/non-dendritic cell hybrids. Yet further, these technologies use a patient's cancerous cell lines and great care would be required in handling and processing these cell lines to avoid further tumorigenesis. Cancerous cell lines are, of course, only available in cancer patients and it is unclear whether these therapies could be applied to patients for whom no immortal cell line is available.

US Patent 5,976,546 to Rockefeller University discloses a method of presenting antigens on the surface of dendritic cells by combining dendritic cells with a complex of (a) a dendritic cell binding protein, (b) a polypeptide antigen and (c) a linker. It is not clear to what extent the presence of a dendritic cell binding protein and linker will affect the ability of the antigen to generate a T-dependent immune response. This disclosure also relates to polypeptide antigens only.

WO 92/21981 describes diagnosis and treatment of inflammatory and degenerative diseases of serous and related tissues. This document discloses a method of treating inflammatory and degenerative diseases of serous and related tissues which comprise the step of administering an immune complex consisting of lamellar bodies or their constituents and antibodies specific thereto.

The first aim of the present invention is to provide a quick and effective method of incorporating molecules into dendritic cells such as to cause dendritic cells and antigen presenting cells to display desired molecules on their surface. The invention relates in particular to dendritic cells and antigen presenting cells modified by this technique. For example, the invention aims to provide a method of incorporating antigens into the surface membranes of dendritic cells so that the antigens are presented to T cells. Alternatively, polynucleic acids coding for antigens may be delivered into dendritic cells for use in known gene therapeutic techniques. Adjuvancy is a phenomenon in which an agent or combination of agents, used together with an antigen, provokes in the host an augmented immunological response. Freund's Complete Adjuvant basically consists of an antigen emulsified in oil with mycobacterial cell walls. It is generally accepted that Freund's adjuvant is the most powerful immuno-stimulant yet discovered. However, its production of tissue granulomata in vaccination has resulted in its discontinued use, not only in man but in experimental animals. This ruling ignores totally the self-evident and established pathological knowledge that the most powerful and long-lasting immune responses in vertebrates derive from organisms or agents which produce a granuloma.

A further aim of the invention disclosed herein is to provide an improved adjuvant, having efficacy approximating that of Freund's Adjuvant but lacking the associated unpleasant side-effects.

In this specification the term "antigen presenting cells" relates to any immune system cells which presents antigens to components of the immune system. This term therefore includes dendritic cells.

The invention is as defined in the claims. According to a first aspect of the present invention there is provided a phospholipid vesicle for producing an immune response, the phospholipid vesicle having an antigen or a polynucleic acid coding for an antigen therein, the phospholipid vesicle being adapted to be phagocytosed by antigen producing cells.

The phospholipid vesicle is multilamellar.

The phospholipid composition of the phospholipid vesicle comprises: phosphatidylcholine 44-60%, sphingomyelin 15-25%, phosphatidylethanolamine 6-10%, phosphatidylserine 2-6%, phosphatidylinositol 2-4%, cholesterol 4-12%.

Preferably, the phospholipid composition of the phospholipid vesicle is: phosphatidylcholine 54%, sphingomyelin 19%, phosphatidylethanolamine 8%, phosphatidylserine 4%, phosphatidylinositol 3%, cholesterol 10%.

Optionally, the phospholipid composition of the phospholipid vesicle includes lysolecithin.

Preferably, the phospholipid composition of the phospholipid vesicle is 0-3% lysolecithin.

Most preferably, the phospholipid composition of the phospholipid vesicle is 2% lysolecithin.

The phospholipid vesicle may be a lamellar body isolated from the mammalian body.

According to a second aspect of the present invention there is provided phospholipid vesicles according to the first aspect for use as a vaccination agent.

According to a third aspect of the present invention there are provided modified antigen presenting cells for inducing a cellular immune response comprising antigen presenting cells isolated from a mammalian body modified by take up of phospholipid vesicle according to the first aspect.

Preferably, the antigen presenting cells are dendritic cells.

According to a fourth aspect of the present invention there is provided a method of incorporating antigens into dendritic cells comprising the step of mixing phospholipid vesicles according to the first aspect of the present invention with dendritic cells.

According to a fifth aspect of the present invention there is provided a pharmaceutical composition for inducing an immune response, the composition comprising phospholipid vesicles according to the first aspect of the present invention or modified dendritic cells according to the second or third aspects of the present invention and a pharmaceutically acceptable carrier.

According to a sixth aspect of the present invention there is provided a protein having a peptide sequence as set forth in sequence ID No. 1 or ID No. 2 and further having a peptide sequence as set forth in sequence ID No. 3 or ID No. 4.

Preferably, the protein acts as an adjuvant when inducing an immune response.

Preferably, the protein may have a peptide sequence that is identical to or homologous to that set forth in sequence ID No. 1 or ID No. 2 and have a further peptide sequence that is identical to or homologous to those set forth in sequence ID No. 3 or ID No. 4 wherein the resulting protein again is able to act as an adjuvant to produce an immune response. Preferably the replacement peptides are 99% homologous.

Optionally the replacements may show between 99% and 75% homology.

Preferably the protein resulting from the homologous peptides has the same or similar activity to the original.

According to a seventh aspect of the present invention there is provided a phospholipid vesicle according to the first aspect of the present invention having therein a protein selected from the group comprising:
(a) a protein according to the sixth aspect.
(b) trehalose dimycolate

According to an eighth aspect of the present invention there is provided a pharmaceutical composition for inducing an immune response comprising phospholipid vesicles according to the seventh aspect, an antigen and a pharmacologically acceptable carrier.

The present invention also provides immuno-stimulant phospholipid vesicles incorporating trehalose dimycolate for controlled micro-granuloma vaccination; phospholipid vesicles incorporating SP-A Peptide Sequence 77-110 for Presenting Antigen to Autologous Professional and Non-Professional APCs *in vitro* to effect an immuno-therapeutic response in a host; and phospholipid vesicles incorporating Trehalose Dimycolate and SP-A Peptide Sequence 77-110.

An example of the present invention will now be illustrated with reference to the following Figures in which:
Figure 1 is a schematic diagram of a process for incorporating material into dendritic cells; and
Figure 2 is a schematic diagram of dendritic cells for use in a pharmaceutical preparation.

Dendritic cells are bone-marrow-derived cells belonging to a different lineage from macrophages. They are characterised by their irregular shape, constitutive expression at high levels of MHC class I and II molecules and a paucity of lysosomes and endocytic vesicles. They are found as veiled cells in afferent lymphatics, as interdigitating cells in T cell areas of secondary lymphoid tissues, and in the thymic medulla (Male et al 1991). It is now established that dendritic cells are the major antigen presenting cell in triggering, with high efficiency, primary T cell-mediated immune responses (Banchereau J, Steinman R.M 1998). Additionally the ability to stimulate primary responses resides in their constitutive expression of a co-stimulatory activity, unlike other antigen presenting cells, which is required in addition to MHC / peptide for activation of resting but previously sensitised T cells. Dendritic cells are widely recognised as being poorly endocytic and doubt remains as to the manner in which antigen is taken up by the cell, although racket-shaped granules, Birbeck granules, are believed by some to endocytose antigenic material and process it for presentation to T cells. Dendritic cells are present as a small percentage of the peripheral blood mononuclear cells (PBMCs) where they circulate to the tissues, migrating from the tissues via the afferent lymph to draining lymph nodes where they form the interdigitating dendritic cells in the paracortex and there present antigen to T cells trafficking through the node. Dendritic cells which pass through the epidermis are known as Langerhans' cells (LCs).

The most recent advance in cancer immunotherapy involves the use of tumour antigens and autologous antigen presenting cells as cancer vaccines (Melman et al. 1998). In clinical trials, *in vitro* pulsing of autologous dendritic cells with antigens expressed by tumour cells have achieved up to 30% partial response and 8% complete response in patients with certain tumours (Tjoa B. et al 1998). Further development of strategies to enhance these promising trials is frustrated by a current knowledge deficit on effective methods or agents which *in vitro* can induce dendritic cells to imbibe the antigen and process the antigenic determinant for presentation on its cell surface, as occurs *in vivo.*

We have, however, discovered that lamellar bodies are phagocytosed by dendritic cells *in vivo.* Lamellar bodies, are produced by most if not all mammalian cells and are released mainly on to the cell surface by exocytotic secretion (3). They serve as cell surface, intercellular and intra-matrix lubricants, surfactants and water repellents. A higher level of production is characteristic of certain specialised tissues involved in providing non-stick surfaces (peritoneum, pericardium, pleura) (Dobbie et al. (1988); Dobbie and Lloyd (1989); Dobbie et al. (1994); Dobbie et al. (1995)), lubrication in locomotion (synovium) (Dobbie et al. (1994); Dobbie et al. (1995)), as a surfactant (lung) or as a water repellent (skin). Lamellar bodies not only pass from the various body cavities directly into the lymphatic system, but in tissues of high secretion they also pass in a retrograde fashion through matrix ground substance and between cells into the draining lymphatics (6).

Lamellar bodies resemble liposomes. However, there are key differences. Lamellar bodies are phospholipid liquid crystals and, in direct contrast to liposomes, are highly flexible since they contain no or low cholesterol. Thus in tissues they constantly form and re-form and, in so-doing, incorporate free, planktonic proteins and peptides from fragmentary proteinaceous debris in the extracellular fluid present in normal host or pathological tissue as in the inflammatory response to invading organisms. Thus lamellar bodies, incorporating both endogenous (self) and exogenous (non-self) material, either exposed on the surface or contained within or between phospholipid bilayers, pass into the lymphatic drainage and are automatically conducted to the loco-regional lympho-reticular tissue. There, on filtering through lymphatic sinuses, lamellar bodies, possessing a bilayer composition more typical of prokaryotic cell membrane (i.e.: bacteria or viruses) are automatically scrutinised by the lymphoid tissue as they have the size, shape and membrane composition of a naked bacterium. In this respect the closest analogy in explanation of the prime function of lamellar bodies is that of universal fly-paper, where intra-tissue self and non-self proteins and peptides are automatically taken up and conducted to the lymphoid tissue. This is the basement tier of the acellular, innate immune system which depends on the physico-chemical interaction between naturally forming and recycling multi-lamellar spheres and planktonic molecules in extracellular fluid. Thus, lamellar bodies are automatic centripetal transporters in the lympho-reticular system. The significance of this basic biological system has hitherto remained unrecognised, in that the ubiquity of lamellar bodies in normal tissues, and their distribution, density and contents in pathological processes have never been observed to our knowledge. Their presence in non-pulmonary tissues are not visualised with the electron microscope unless the tissues are deliberately fixed and processed in a manner which specifically preserves their delicate ultrastructure which led to their discovery in lung (Refs).

The present invention relates to man-made lamellar body like constructs, which are phospholipid vesicles designed to mimic natural lamellar bodies and into which chosen antigens can be inserted. The invention also relates to the method by which they can be incorporated into dendritic cells and to the dendritic cells which are thereby formed. These dendritic cells containing chosen antigens can then, in an otherwise known method, be used in pharmaceutical preparations for the treatment of a broad range of conditions.

The phospholipid vesicles of the present invention are constructs consisting of phospholipids natural to vertebrates, formed as a multilamellar liposome which closely reflects the chemical composition and ultrastructural disposition of lamellar bodies. These occur in varying density in most tissues throughout the body in mammals e.g.: lung, synovium, peritoneum, pleura and pericardium (Dobbie (1988); Dobbie et al. (1988); Dobbie and Lloyd (1989); Dobbie et al. (1994); Dobbie et al. (1995)). They also have been found in amphibia, teleosts and elasmobranchs (Dobbie and Lewis, 1999, unpublished data). Lamellar bodies are characterised by phospholipid bilayers, usually of regular periodicity (Dobbie (1989); Dobbie et al. (1994)). In contrast to the composition of the phospholipid bilayers of normal mammalian cell membranes which contain significant amounts of cholesterol, lamellar bodies have low concentrations or no cholesterol in their lamellae. In this respect lamellar bodies are closer to the compositional characteristics and ultrastructural disposition of the cell membrane of naked micro-organisms, bacteria and viruses.

The invention involves construction of multi-lamellar liposomes of chemical composition similar or close to lamellar bodies which incorporates antigenic material, protein, peptides or other molecules extruding from the surface or present within and between the phospholipid bilayers.

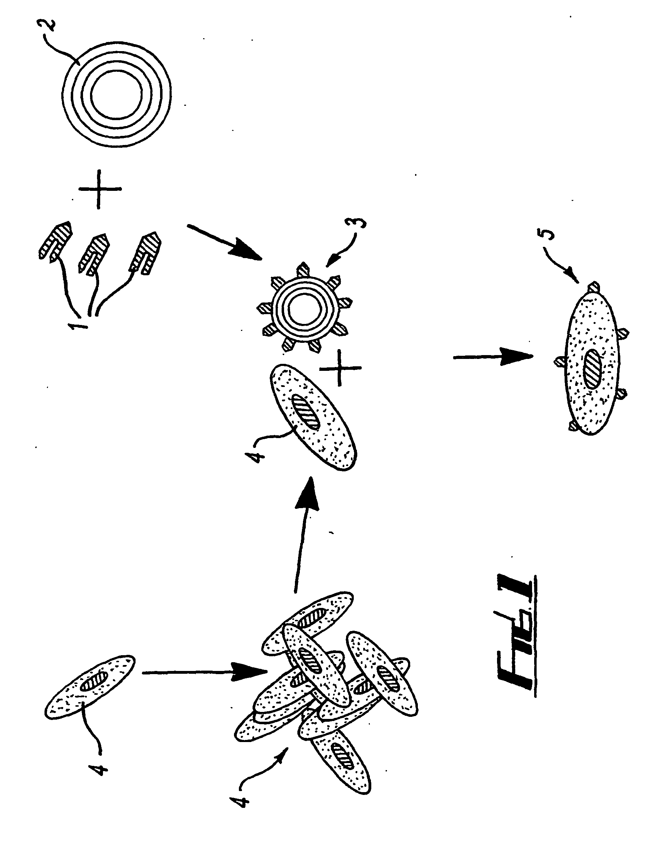

Figure 1 shows a schematic diagram of how this procedure is carried out. Antigens 1 are first incorporated into a lamellar-body-like phospholipid vesicle 2, forming an antigen-loaded phospholipid vesicle 3.

The phospholipid vesicle so constructed is used as the carrier of a selected antigen for presentation to a professional (dendritic cell or macrophage), or to a non-professional antigen-presenting cell (antigen presenting cell). These cells internalise the phospholipid vesicle together with the antigenic material distributed throughout its various compartments. The antigenic material is then processed intra-cellularly by the antigen presenting cell and the antigenic determinant is subsequently presented on the cell surface with the appropriate, accompanying, identifying and stimulatory surface proteins e.g.: MHC 1 and 2 molecules.

In this example, a patient's own dendritic cells 4 are cultured *in vitro* and combined with phospholipid vesicles to yield modified dendritic cells 5. Although this description relates to dendritic cells in particular, it will be clear to one skilled in the art that the same principle can be applied to modify other antigen presenting cells.

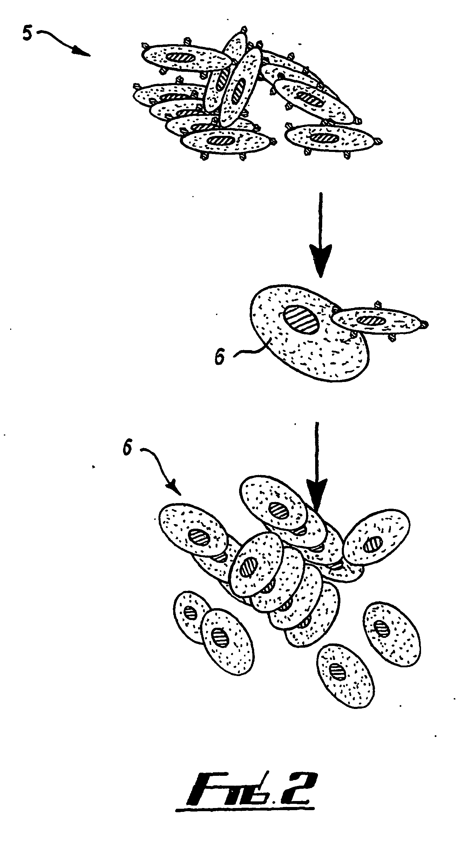

Figure 2 shows in figurative form a plurality of modified dendritic cells 5. In the therapy, autologous dendritic cells, isolated from peripheral blood, cultured in vitro and primed with the antigen delivered by the phospholipid vesicle, when returned to the patient by whatever route (for example, injection into the lymphatic system), proceed to the lympho-reticular system where they stimulate production of antigen-specific T lymphocytes 6 which constitute the effector cell in completing the cellular immune response to body cells bearing the selected antigen.

### Lamellar Bodies

Natural liposomes, lamellar bodies, are produced by most if not all mammalian cells and are released mainly on to the cell surface by exocytotic secretion (Dobbie (1989)). They serve as cell surface, intercellular and intra-matrix lubricants, surfactants and water repellents . A higher level of production is characteristic of certain specialised tissues involved in providing non-stick surfaces (peritoneum, pericardium, pleura) (Dobbie et al. (1988); Dobbie and Lloyd (1989); Dobbie et al. (1994); Dobbie et al. (1995)), lubrication in locomotion (synovium) (Dobbie et al. (1994); Dobbie et al. (1995)), as a surfactant (lung) or as a water repellent (skin). Lamellar bodies not only pass from the various body cavities directly into the lymphatic system, but in tissues of high secretion they also pass in a retrograde fashion through matrix ground substance and between cells into the draining lymphatics (Dobbie and Anderson (1996)).

### Structure of phospholipid vesicles.

In this invention phospholipid vesicles are constructed using specific phospholipids in proportions similar to those found in lamellar bodies in normal tissues. The key feature which distinguishes the phospholipid vesicles described in the present Application from liposomes is their low content or absence of cholesterol. In biomedical applications liposomes, as synthetic constructs, are primarily designed for compartmental containment and preservation of pharmaceuticals and diverse agents. Thus they are constructed with high levels of cholesterol which confer a membrane stability and low porosity, mimicking mammalian cell membranes. Therefore it follows that the bilayer concentration of cholesterol is the key determinant of the circulatory half-life for liposomes designed as drug carriers. The inhibitory effect of cholesterol on the up-take of liposomes by the lympho-reticular system, as measured in liver and spleen, is well-established (Patell HM et al. 1983). In direct contrast, phospholipid vesicles, modelled on the properties of lamellar bodies, are readily taken up by phagocytic cells and as in the case of liposomes with low cholesterol content , are rapidly removed from circulation by lympho-reticular tissue.

The principle phospholipid constituents of lamellar bodies are phosphatidylcholine (PC), sphingomyelin (SPH), phosphatidylethanolamine (PE), phosphatidylserine (PS), phosphatidylinositol (PI) and lysolecithin (LPC). The phospholipid composition of lamellar bodies shows slight variation according to the cell of origin.

PC is the principle phospholipid in lamellar bodies, irrespective of site of origin. The percentage PC concentration varies from around 70% in lung lavage to 45% in synovial fluid (Refs) The next phospholipid in ranking concentration is SPH (5-15%). Thereafter, PE, PS, PI, PG and LPC are present in varying, single digit percentage concentrations in lamellar bodies according to site of origin.

The preferred composition of phospholipids and cholesterol for phospholipid vesicles comprises: PC 54%: SPH 19%: PE 8%: PS 4%: PI 3%: cholesterol 10%. These values are median and the following range of compositions have been found in natural lamellar bodies (private research): PC 44-60%, SPH 15-23%, PE 6-10%, PS 2-6%, PI 2-4%, Cholesterol 4-12%. These figures are percentage by weight.

LPC may also be incorporated into the vesicles at 2% by weight which follows the range found in natural lamellar bodies of 0-3%.

Phospholipid vesicles in the form of liposomes are, of course, well known. However, liposomes are made by those skilled in the art with high cholesterol concentrations to improve their rigidity. Liposomes containing cholesterol at 20% or below would be considered to be cholesterol poor (Love WG et al, 1990). Liposomes incorporating a high ratio (50%) of cholesterol, where it is equimolar with the phospholipids, have a highly stable structure (Kirby et al, 1980, Senior et al, 1982) and so, until this invention, it would not to our knowledge have been obvious to try using low-cholesterol vesicles. The cholesterol content of lamellar bodies derived from pulmonary alveoli has been found to contain around 10% cholesterol (Schmitz G, Muller J 1991) (J Lipid Research. 32:1539).

The presence of sphingomyelin in natural lamellar bodies and in the phospholipid vesicles claimed in the present invention is important. Sphingomyelin is not generally used, to our knowledge, in liposomes and serves to give flexibility and softness to lamellar bodies. Conventional liposome technology teaches that rigidity is better for the delivery of chemicals; however, we have found that flexible, low-cholesterol, sphingomyelin containing phospholipid vesicles are ideal for delivery of antigen to antigen presenting cells.

### Reparation of Phospholipid vesicles

Phospholipid vesicles are prepared by a technique similar to that used to produce hand-shaken multi-lamellar vesicles (New RRC, 1990). The phospholipid mixture, together with cholesterol in the percentages given by weight are dissolved in a chloroform / methanol solvent mixture (2:1 vol/vol). The lipid solution is introduced into a round-bottomed flask and attached to a rotary evaporator. The flask is evacuated and rotated at 60 r.p.m. in a thermostatically controlled waterbath at a temperature of 30°C until a dry lipid film is deposited. Nitrogen is introduced into the flask and the residual solvent is removed before its connection to a lyophilizer where it is subjected to a high vacuum at room temperature for one hour. After release of the vacuum and following flushing with nitrogen, saline containing solutes (selected antigen) for entrapment is added. The lipid is hydrated within the flask, flushed with nitrogen, attached to the evaporator, and rotated at 60 r.p.m. at room temperature for thirty minutes. The suspension is allowed to stand for two hours at room temperature to complete the swelling process.

### Antigen-loaded Phospholipid vesicles

The antigen to be presented (protein, peptide or other antigenic agent) to the professional antigen presenting cell, e.g. dendritic cell, is prepared as a solute in the normal saline (0.9%) used in the hydration and production of the phospholipid vesicles. Like liposomes, the phospholipid vesicles of the present invention cannot be sterilised by exposure to high temperatures and are also sensitive to various types of radiation and chemical sterilising agents. For human use every stage in their production must be carried out under aseptic conditions with the initial organic solution of lipids being passed through membrane filters of regenerated cellulose (pore size 0.45µm) and glass fibre, before drying down, to remove micro-organisms, spores and pyrogenic material (Ref. RRC New, p 103). Since the phospholipid vesicles disclosed herein are similar to the multi-lamellar vesicle-type of liposome, they are mechanically stable upon storage for long periods of time. Their size can be regulated by extrusion.

The phospholipid vesicles of the present invention are similar in construction to multilamellar vesicle type liposomes which, in contrast to other types of liposomes, give a much more gradual and sustained release of material (RRC New, p28). This property is important in the release of antigen, both extra and intra-cellularly

Thus prepared, the phospholipid vesicles can be used to present antigen to professional and non-professional antigen presenting cells.

It is known at the present time to deliver nucleic acids into antigen presenting cells, such nucleic acids then becoming incorporated into the cells and therein expressing proteins which are presented on the surface of the cells. In an alternative embodiment, phospholipid vesicles are made with RNA or DNA therein and, upon take up of the phospholipid vesicles, deliver the nucleic acids into antigen presenting cells, acting as a convenient gene therapy agent.

### Separation, Isolation and Culture of Human Dendritic cells

PMBCs derived either from 50ml of freshly-drawn venous blood or by leucapharesis are isolated using hypaque density gradient centrifugation under sterile conditions. The PMBCs are re-suspended in complete medium (OPT1MEM medium) in 5% heat-inactivated autologous plasma and plated in a 75cm2 tissue culture flask with 2 - 3 x 10' cells per flask The cell suspensions are incubated in a humidified incubator (37°C', 5% C02) for 60 mins. The non-adherent cells are removed and the adherent cells are washed gently with warm (37°C) complete medium. Dendritic cell propagation medium (dendritic cellPM: complete medium, 500 units/ml granulocyte macrophage-colony stimulating factor (GM-CSF) and 500 units /ml Interleukin 4 (IL-4) are added to the adherent cells (10ml flasks) and cultured for 4-6 days.

### Modification of Cultured Autologous dendritic cells with Antigen-Loaded Phospholipid vesicles

Antigen-loaded phospholipid vesicles, suspended in a minimal volume of normal (0.9%) saline, are added to the culture flasks, exposing them to the cultured dendritic cells for 1 hour at 37° C. Thereafter the cells are washed gently in PBS and re-suspended in normal saline for intravenous or intra-lymphatic intra-dermal administration to the patient.

### Adjuvancy

We have found that "foamy" debris and the contents of foam cells, both mononuclear and multinuclear, a characteristic feature of all granulomata, are revealed as lamellar bodies when the tissues are fixed in tannic acid glutaraldehyde fixative and can now provide a satisfactory explanation of the nature of vaccination by Freund's Complete Adjuvant.

Injection of Freund's Complete Adjuvant with antigen creates an artificial depot of antigen through the induction of a self-generating and self-maintaining natural depot through the formation of a lamellar body containing granuloma. This results from the inhibition of phagosome/lysosome fusion by trehalose dimycolate (derived from mycobacterial cell walls) which causes intracellular accumulation of lamellar bodies. Macrophage capacity to degrade lamellar bodies is further reduced by phagocytosed mineral oil droplets and a foreign body reaction is induced. Cells replete with lamellar bodies which they are unable to digest through blockage of phagosome/lysosome fusion, undergo necrosis, releasing into the developing granuloma an escalating load of lamellar bodies which, on being re-phagocytosed by remaining viable cells, are in turn blocked by the local effect of the Freund's adjuvant.

Freund's Complete Adjuvant therefore creates a granuloma which mimics the effect of a natural mycobacterial granuloma, where live intracellular bacilli release trehalose dimycolate, blocking all phagosome/lysosomal fusions.

In a vaccine containing Freund's Complete Adjuvant, the chosen antigen is automatically taken up into lamellar bodies from the extracellular fluid at the injection site. Thus the antigen is concentrated locally as the self-generating and self-maintaining depot where the antigen molecules are sequentially dispersed throughout an increasing number of forming and re-forming lamellar bodies. Of organismal dimension and with a phospholipid composition (low cholesterol) resembling a bacterial cell membrane, lamellar bodies are avidly phagocytosed by the *concentric rings* of leucocytes which envelop the granuloma.

This is a hitherto unrecognised, innate defence mechanism in vertebrates, where phagocytosed organisms, in their own defence, through blocking intracellular phagosome/lysosomal fusion, inevitably lead to local accumulation of large numbers of lamellar bodies bearing a selection of antigenic portions of the dead invaders. This automatically creates an escalating amplification of local, then distant, exposure to antigenic molecules borne by the lamellar bodies, and ultimately results in a powerful therapeutic response to even the most virulent of organisms.

### Mesosome-Lamellar Body Interaction in the Presentation of Bacterial Antigens in Granulomata.

Revealed by similar electron microscopy techniques as have disclosed lamellar bodies in animal tissue, mesosomes are small spheres (0.05um), composed of oligo-lamellar phospholipid bilayers which are present in most bacteria. In living bacteria they can be found in a variety of para-cell membrane and para-septal locations. Although they have been ascribed various functions, such as intercellular transfer of DNA, their nature and function have excited little curiosity. It is recognised however, that damaged, dying and dead organisms release numerous mesosomes which disperse in the debris of a local inflammatory event.

Mesosomes are therefore identical in structure to liposomes and lamellar bodies, having a very small radius of curvature and composed entirely of phospholipids (cholesterol free). It is established that such liposomes, because of the high intra-membrane tension, immediately fuse with larger liposomes in any mixture of liposomes of varying size (RRC New 1991. P28). Thus it follows that mesosomes released from dying bacteria will, through their physical chemistry, be rapidly incorporated into lamellar bodies congregating in the locality of the acute inflammatory event. Phospholipid bilayers of bacterial origin, whether derived from mesosomes or disintegrating bacterial cell membrane, and bearing potentially antigenic protein or peptide material, will be automatically incorporated at that site into the phospholipid bilayers of the larger "liposomes" i.e. lamellar bodies, present in the mixture.

The principle ingredient of Freund's Complete Adjuvant, emulsified mycobacterial cell wall, unwittingly utilises this hitherto unrecognised phenomenon which occurs at the site of inflammatory response to invading organisms.

The phospholipid vesicles of the present invention, through their size, composition (low cholesterol) and antigen loading, thus mimic this phenomenon.

### Heat Shock (Stress Proteins

The production of heat shock proteins (HSPs) is the universal response of prokaryotic and eukaryotic cells to the damaging effect of heat (Ritossa F 1962. Kaufmann SHE 1990). Initially it was believed that HSPs had evolved for the prevention of unfolding of proteins at high temperature, it was subsequently shown that many insults other than heat induced HSP synthesis, such that the term "stress proteins" was introduced. It is now clear that HSPs also serve important physiological functions, and that many are present and active in normal cells (Ellis J. 1987. Pelham H 1988). Thus the designation "molecular chaperone" was coined to account for their more general role as house-keeping proteins in the cell (Ellis 1987, Pelham 1988). Among the most conserved proteins known in phylogeny, it is therefore not surprising that HSPs are also deeply involved in immunity (Kaufman 1990).

The fact that pathogen and host use similar mechanisms to protect themselves from each other, and that this is achieved by similar molecules, fits well into the broad realm of HSP function. That a single molecule comprises epitopes with potential relevance to protection adjacent to epitopes with potential relevance to pathogenesis, is believed to be a significant factor in the immunogenicity of these proteins (Kaufman 1990).

### Linkage of Surfactant Protein A (SP-A) and 65 kDa Mycobacterial HSP

There is diverse evidence that in man mycobacterial HSPs are a highly immunogenic molecular species in the host defence against mycobacterial infection (tuberculosis, leprosy), and considerable circumstantial evidence that in genetically susceptible individuals they play a pivotal role in auto-immunity (inflammatory joint disease) (Dobbie et al 1994).

Until recently believed to be exclusively associated with the production, dispersion and metabolism of pulmonary surfactant, SP-A, a constitutive secretory protein of Type II pneumocytes, surprisingly has been shown to be present in abundance in many body tissues. Initially described as an apoprotein intimately attached to alveolar lamellar bodies, it is now recognised that it is invariably found in high concentrations in extra-pulmonary tissues which specialise in secretion of lamellar bodies (Dobbie et al). Thus SP-A has been demonstrated in synovium, peritoneum, pericardium, pleura, skin, terminal ileum, glandular ducts (biliary, lacrimal, salivary), gall bladder and prostate (Dobbie 1994). It is noteworthy that SP-A shows considerable homology with the Clq component of the Complement Cascade.

Mycobacteria are phospholipid-nutrient obligates. It is therefore not coincidental that tissues which secrete, as lamellar bodies, the highest amount of phospholipid (Dobbie & Pavlina), are those tissues (lung, joints, serosal cavities, terminal ileum, etc.) subject to primary infection by pathogenic mycobacteria. Mycobacterial infections in man therefore occur at sites of high SP-A production.

A striking link between SP-A and mycobacterial HSPs has recently been demonstrated through co-localisation of antibodies to SP-A (PE 10, 5B8, 8H10) and mycobacterial HSPs (ML30) in cells secreting lamellar bodies through immuno-staining. (Dobbie et al. 1994). This linkage is further demonstrated in Dot Blot testing that ML30 reacted with SP-A, a pure preparation isolated on a sepharose immune-affinity column, coupled to the monoclonal antibodies 5B8 and 8H10. This indicates shared epitopes, i.e. homologous amino acid sequences, between SP-A and mycobacterial HSPs. This would be a reasonable strategy on the part of mycobacterial pathogens, in that their HSPs achieved relative camouflage with sequences common to SP-A, since they were obliged to live in phospholipid-rich areas of the host.

It is established that alveolar macrophages are the principle cells involved in phagocytosing and re-cycling alveolar phospholipid. Furthermore it is established that SP-A is crucial to this process while spiking of liposomes with SP-A increases the rate of uptake of liposomes in Type II pneumocyte cell cultures. We have shown that macrophages at other extra-pulmonary sites of high level lamellar body production (joints and body cavities) are also the main cells involved in lamellar body uptake and recycling of phospholipids. SP-A has therefore been demonstrated by immuno-staining in significant concentrations in macrophages at those extra-pulmonary locations. SP-A has therefore been shown to facilitate the uptake of lamellar bodies by at least one of the professional APCs.

We therefore conclude that this linkage between SP-A and mycobacterial HSPs represents a powerful immuno-stimulant involving highly conserved eukaryotic and prokaryotic proteins in intimate reciprocal relationship between simple phospholipid, automatically forming and re-forming lamellar bodies and bacterial mesosomes. It is further suggested that this mechanism in genetically susceptible, immunologically dysfunctional individuals is responsible for the triggering of certain forms of auto-immunity, associated with granuloma formation in which HSPs have been recently presented as candidate target auto-antigens in inflammatory joint disease.

### Amino Acid (Aa) Sequence Comparison between Lamellar Body-Associated Human SP-A and Mycobacterial HSPs.

Comparison of sequences from human lung lamellar body SPA with M leprae 65kDa HSP were made using multiple span comparisons and Dayhoffs scores to weight Aa match. The minimal length for selecting autologous peptides was 10 Aa, with two possibilities - no deletion or deletion of equal to or less than 3 Aas with aligned homologous peptides. SP-A has 150 Aa, HSP 65 kDa has 540 Aa. As a second step, alignments exhibiting potentially antigenic sub-sequences were selected. Two criteria were used to make the selection: the presence inside the peptide of an amphipaticity compatible with an alpha helix and the presence of one of the two patterns observed in well-determined antigenic peptides. These two patterns correspond to preferred sequences of Aas based on their hydrophilic, hydrophobic or neutral character.

Sequences of the 65kDa antigen and SP-A, with no deletion, which show homology are given below:

| | | | |
|---|---|---|---|
| M leprae | (164) | R G I E K A V E K V T E | (175) |
| SP-A | (121) | + + K + 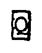 C + + M 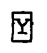 + 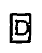 | (132) |

| | | | |
|---|---|---|---|
| + = identity 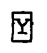 = Aa with identical physico-chemical characteristics | | | |

This sequence of the 65 kDa antigen has been recognised as an epitope by anti-M leprae monoclonal antibodies (H 9) (Shinnick et al 1987). Synthetic peptides of this sequence have been identified as an epitope involved in human T cell recognition of M tuberculosis (Lamb et al 1989).

A second sequence exhibiting homology is shown below:

| | | | |
|---|---|---|---|
| M leprae | (175) | V E E S N T F G L Q L E L T E G | (191) |
| SP-A | (80) | + K 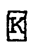 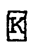 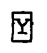 + + 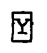 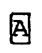 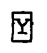 * 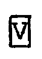 G + + + + | (94) |

| | | | |
|---|---|---|---|
| + = identify 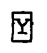 = Aa with identical physico-chemical characteristics * = deletion | | | |

Patients with rheumatoid arthritis demonstrate specific T-lymphocyte reactivity to this mycobacterial epitope (van Eden 1988).

### Role of HSPs in Antigen Capture and as Chaperones in Dendritic Cells

Evidence is accumulating that host HSPs have an important role in initiating and amplifying the immune response through their effect on professional APCs, especially on DCs (Colaco, 1998). There is increasing interest in the immuno-stimulant effect of endogenous HSPs, provoked by early release of cytokines in facilitating the capture of antigen by DCs. Furthermore, endogenous HSPs are increasingly recognised to act as chaperones, channelling antigens into appropriate intracellular cisternae and organelles for efficient processing of the antigenic determinant.

Our analysis of published data on the functioning of Freund's Complete Adjuvant indicates that mycobacterial HSPs provoke in man innate, rapid and powerful immune stimuli through the mechanisms previously detailed. Thus mycobacterial HSPs or derived peptides, adherent to or incorporated in lamellar bodies, act to facilitate both their attachment and phagocytosis.

It is further maintained that peptide motifs, including those Aa sequences shared by 65kDa, mycobacterial HSPs and human SP-A provide the pivotal peptides which drive this effective antigen capture and processing by DCs. Therefore it is proposed that lamellar body-like phospholipid vesicles, loaded with the said motifs and a selected antigen, will utilise this mycobacterial HSP immuno-stimulant or adjuvant property in pulsing autologous DCs in vaccination or in immuno-therapy.

Thus the key feature of this invention is the addition of peptides which exploit an innate, early and powerful response by APCs, where a motif(s) of a species-recognised dangerous pathogen i.e. mycobacterial HSP peptides, guarantees immediate attention and capture of antigen when presented in association with these motifs. Analysis implies that the high efficiency of Freund's Complete Adjuvant, without all of the accompanying mycobacterial debris and oil droplets, will be obtained through the incorporation of these motifs in phospholipid vesicles together with loaded antigen.

### Composition of Phospholipid vesicles primed with Mycobacterial ESP / SP-A Motifs

Phospholipid vesicles are composed of phospholipids and cholesterol in the ratios specified above. The Aa motifs are present as the solute (listed concentrations) in the normal (0.9%) saline used in the hydration process in the formation of the phospholipid vesicles.

### Immune-Stimulant Phospholipid vesicles incorporating Trehalose Dimycolate for Controlled Micro-Granuloma Vaccination

The present invention also provides immuno-stimulant phospholipid vesicles incorporating trehalose dimycolate for controlled micro-granuloma vaccination; phospholipid vesicles incorporating SP-A Peptide Sequence 77-110 for Presenting Antigen to Autologous Professional and Non-Professional APCs *in vitro* to Effect an ImmunoTherapeutic Response in a Host; and phospholipid vesicles Incorporating Trehalose Dimycolate and SP-A Peptide Sequence 77-110.

### Documents referenced herein

Dobbie JW (1988) Ultrastructural similarities between mesothelium and Type II pneumocytes and their relevance to phospholipid surfactant production by the peritoneum. In: Advances in Continuous Ambulatory Peritoneal Dialysis, (Eds: Khanna R, Nolph KD, Prowant B), University of Toronto Press, Toronto, pp. 47-53.

Dobbie JW, Pavlina T, Lloyd J, Johnson RC (1988) Phosphatidylcholine synthesis by peritoneal mesothelium: Its implications for peritoneal dialysis, Am.J. Kid. Dis. 12,31-36.

Dobbie JW, Lloyd JK (1989) Mesothelium secretes lamellar bodies in a similar manner to Type II pneumocyte secretion of surfactant. Perit. Dial. Int. 9: 215-219.

Dobbie JW, Tasiaux N, Meijers P et al. (1994) Lamellar bodies in synoviocytes, mesothelium and specific epithelia as possible site of auto-antigen in rheumatoid disease. B J Rheum 33:508-519.

Dobbie JW, Hind C, Meijers P, Bodart C, Tasiaux N, Perret J, Anderson JD. (1995) Lamellar body secretion: Ultrastructural analysis of an unexplored function of synoviocytes. Br J Rheum. 34:13-23.

Dobbie JW, Anderson JD (1996) Infrastructure, distribution and density of lamellar bodies in human peritoneum. Perit Dial Int. 16:482-487.

New RRC ed. (1990) Liposomes: A Practical Approach. Oxford University Press, New York. pp 36-39.

Male D, Champion B, Cooke A, Owen M. (1991) In: Advanced Immunology. Second Edition. Gower Medical Publishing, London, New York. pp 8.13-8.14.

Melman I, Turley SJ, Steinman RM. (1998) Antigen processing for amateurs and professionals. Trends Cell Biol. 8:231-35.

Tjoa B Simmons S. Bowes V. et al. (1998) Evaluation of Phase I/II clinical trials in prostate cancer with dendritic cells and PSMA peptides. Prostate 36:39-44.

Banchereau J, Steinman RM 1998 Dendritic cells and the control of immunity. Nature 392:245.

Colaco CALS (1998) Towards a unified theory of immunity: Dendritic cells, stress proteins and antigen capture. Cell Mol Biol. 44:883-90.

Markowicz S, Engelman EG (1990) Granulocyte-macrophage colony-stimulating factor promotes differentiation and survival of human peripheral blood dendritic cells in vitro. J Clin Invest. 85:955-61.

RitossaF (1962) Experientia 18:571-73.

Kaufmann SHE (1990) Heat shock proteins and the immune response. Immunology Today, 1 1:129-36).

Ellis J. (1987) Nature 328:378-79.

Pelham H (1988) Nature 332:776-77.

## Claims

1. A phospholipid vesicle for producing an immune response, the phospholipid vesicle having a multilamellar, lamellar-body like, structure and the phospholipid composition of the phospholipid vesicle comprising :
phosphatidylcholine 44% to 60%,
sphingomyelin 15% to 25%,
phosphatidylethanolamine 6% to 10%,
phosphatidylserine 2% to 6%,
phosphatidylinositol 2% to 4%
and cholesterol 4% to 12%
wherein the figures are percentage by weight, the phospholipid vesicle having an exogenous antigen or a polynucleic acid coding for an antigen therein, the phospholipid vesicle being adapted to be phagocytosed by antigen presenting cells.

2. A phospholipid vesicle as claimed in Claim 1 wherein the phospholipid composition comprises
phosphatidylcholine 54%,
sphingomyelin 19%,
phosphatidylethanolamine 8%,
phosphatidylserine 4%,
phosphatidylinositol 3%
and cholesterol 10%
wherein the figures are percentage by weight.

3. A phospholipid vesicle as claimed in any of the previous Claims, wherein the phospholipid composition includes lysolecithin.

4. A phospholipid vesicle as claimed in Claim 3, wherein the phospholipid composition includes 0% to 3% by weight lysolecithin.

5. A phospholipid vesicle as claimed in Claim 3 or Claim 4, wherein the phospholipid composition of the vesicle includes 2% lysolecithin.

6. A phospholipid vesicle as claimed in Claim 3 or Claim 4, wherein the vesicle is a lamellar body isolated from the mammalian body.

7. A phospholipid vesicle as claimed in any one of the previous Claims, for use as a vaccination agent.

8. A phospholipid vesicle as claimed in Claims 1 to 7, having therein a protein having a peptide sequence that is homologous or identical to that set forth in sequence ID No 1 or No 2, and having a further peptide sequence that is homologous or identical to those set forth in sequence ID No 3 or No 4, wherein the resulting protein is able to act as an adjuvant to produce an immune response.

9. A phospholipid vesicle as claimed in Claim 8, wherein the peptide sequence that is homologous shows
between 99% and 75% homology to SEQ ID No 1, SEQ ID No 2, SEQ ID No 3 or SEQ ID No 4 respectively.

10. A phospholipid vesicle as claimed in Claim 8, wherein the peptide sequence that is homologous shows 99% homology to SEQ ID No 1, SEQ ID No 2, SEQ ID No 3 or SEQ ID No 4 respectively.

11. A phospholipid vesicle according to Claim 8 having therein a protein having a peptide sequence as set forth in sequence ID No 1 or ID No 2, and further having a peptide sequence as set forth in sequence ID No 3 or ID No 4.

12. A phospholipid vesicle as claimed in any one of Claims 1 to 7, having therein trehalose dimycolate.

13. A phospholipid vesicle as claimed in any one of Claims 1 to 7 incorporating SP-A peptide sequence 77 to 110 for presenting antigen to autologist professional and non-professional antigen presenting cells in vitro to effect an immunotherapeutic response in a host.

14. A phospholipid vesicle as claimed in any one of Claims 1 to 7 incorporating trehalose dimycolate and SP-A peptide sequence 77 to 110.

15. Modified antigen presenting cells for inducing a cellular immune response comprising antigen presenting cells isolated from a mammalian body, modified by take-up of phospholipid vesicles according to any one of Claims 1 to 12.

16. Modified antigen presenting cells as claimed in Claim 15, wherein the antigen presenting cells are dendritic cells.

17. A method of incorporating antigens into dendritic cells, comprising the step of mixing phospholipid vesicles according to Claims 1 to 7 with dendritic cells.

18. A pharmaceutical composition for inducing an immune response comprising a phospholipid vesicle according to Claims 1 to 7, or modified antigen presenting cells according to Claims 15 and 16, and pharmacologically acceptable carrier.

## Patentansprüche

1. Ein Phospholipidvesikel zum Erzeugen einer Immunreaktion, wobei das Phospholipidvesikel eine multilamellare, einem Lamellarkörper ähnliche Struktur aufweist und die Phospholipidzusammensetzung des Phospholipidvesikels Folgendes beinhaltet:
44 % bis 60 % Phosphatidylcholin,
15 % bis 25 % Sphingomyelin,
6 % bis 10 % Phosphatidylethanolamin,
2 % bis 6 % Phosphatidylserin,
2 % bis 4 % Phosphatidylinositol
und 4 % bis 12 % Cholesterin,
wobei die Zahlen Massenanteile sind, wobei das Phospholipidvesikel ein exogenes Antigen oder eine Polynucleinsäure, die ein Antigen codiert, darin aufweist, wobei das Phospholipidvesikel angepasst ist, um von antigenpräsentierenden Zellen durch Phagozytose aufgenommen zu werden.

2. Phospholipidvesikel gemäß Anspruch 1, wobei die Phospholipidzusammensetzung Folgendes beinhaltet
54 % Phosphatidylcholin,
19 % Sphingomyelin,
8 % Phosphatidylethanolamin,
4 % Phosphatidylserin,
3 % Phosphatidylinositol
und 10 % Cholesterin,
wobei die Zahlen Massenanteile sind.

3. Phospholipidvesikel gemäß einem der vorhergehenden Ansprüche, wobei die Phospholipidzusammensetzung Lysolecithin umfasst.

4. Phospholipidvesikel gemäß Anspruch 3, wobei die Phospholipidzusammensetzung 0 bis 3 % Massenanteil Lysolecithin umfasst.

5. Phospholipidvesikel gemäß Anspruch 3 oder Anspruch 4, wobei die Phospholipidzusammensetzung des Vesikels 2 % Lysolecithin umfasst.

6. Phospholipidvesikel gemäß Anspruch 3 oder Anspruch 4, wobei das Vesikel ein aus dem Säugetierkörper isolierter Lamellarkörper ist.

7. Phospholipidvesikel gemäß einem der vorhergehenden Ansprüche zur Verwendung als ein Mittel zur Impfung.

8. Phospholipidvesikel gemäß den Ansprüchen 1 bis 7, das darin ein Protein aufweist, das eine Peptidsequenz aufweist, die zu der in SEQ ID NO 1 oder SEQ ID NO 2 dargelegten homolog oder identisch ist, und eine weitere Peptidsequenz aufweist, die zu den in SEQ ID NO 3 oder SEQ ID NO 4 dargelegten homolog oder identisch ist, wobei das resultierende Protein als ein Adjuvans zum Erzeugen einer Immunreaktion wirken kann.

9. Phospholipidvesikel gemäß Anspruch 8, wobei die Peptidsequenz, die homolog ist, zwischen 99 % und 75 % Homologie zu SEQ ID NO 1, SEQ ID NO 2, SEQ ID NO 3 bzw. SEQ ID NO 4 zeigt.

10. Phospholipidvesikel gemäß Anspruch 8, wobei die Peptidsequenz, die homolog ist, 99 % Homologie zu SEQ ID NO 1, SEQ ID NO 2, SEQ ID NO 3 bzw. SEQ ID NO 4 zeigt.

11. Phospholipidvesikel gemäß Anspruch 8, das darin ein Protein aufweist, das eine Peptidsequenz wie in SEQ ID NO 1 oder SEQ ID NO 2 dargelegt aufweist und ferner eine Peptidsequenz wie in SEQ ID NO 3 oder SEQ ID NO 4 dargelegt aufweist.

12. Phospholipidvesikel gemäß einem der Ansprüche 1 bis 7, das darin Trehalosedimycolat aufweist.

13. Phospholipidvesikel gemäß einem der Ansprüche 1 bis 7, das SP-A-Peptidsequenz 77 bis 110 zum In-vitro-Präsentieren von Antigen für autologe, professionelle und nicht professionelle antigenpräsentierende Zellen einschließt, um eine immuntherapeutische Reaktion in einem Wirt zu bewirken.

14. Phospholipidvesikel gemäß einem der Ansprüche 1 bis 7, das Trehalosedimycolat und SP-A-Peptidsequenzen 77 bis 110 einschließt.

15. Modifizierte antigenpräsentierende Zellen zum Induzieren einer zellulären Immunreaktion, die aus einem Säugetierkörper isolierte antigenpräsentierende Zellen, modifiziert durch die Aufnahme von Phospholipidvesikeln gemäß einem der Ansprüche 1 bis 12, beinhalten.

16. Modifizierte antigenpräsentierende Zellen gemäß Anspruch 15, wobei die antigenpräsentierenden Zellen dendritische Zellen sind.

17. Ein Verfahren zum Einschließen von Antigenen in dendritische Zellen, das den Schritt des Mischens von Phospholipidvesikeln gemäß den Ansprüchen 1 bis 7 mit dendritischen Zellen beinhaltet.

18. Eine pharmazeutische Zusammensetzung zum Induzieren einer Immunreaktion, die ein Phospholipidvesikel gemäß den Ansprüchen 1 bis 7 oder modifizierte antigenpräsentierende Zellen gemäß den Ansprüchen 15 und 16 und einen pharmakologisch zulässigen Träger beinhaltet.

## Revendications

1. Une vésicule phospholipidique pour produire une réponse immunitaire, la vésicule phospholipidique ayant une structure multilamellaire semblable à un corps lamellaire et la composition phospholipidique de la vésicule phospholipidique comprenant :
phosphatidylcholine : 44 % à 60 %,
sphingomyéline : 15 % à 25 %,
phosphatidyléthanolamine : 6 % à 10 %,
phosphatidylsérine : 2 % à 6 %,
phosphatidylinositol : 2 % à 4 %
et cholestérol : 4 % à 12 %
dans laquelle les chiffres sont des pourcentages en poids, la vésicule phospholipidique ayant dans celle-ci un antigène exogène ou un acide polynucléique codant pour un antigène, la vésicule phospholipidique étant adaptée pour être phagocytée par des cellules présentatrices d'antigènes.

2. Une vésicule phospholipidique telle que revendiquée dans la revendication 1 dans laquelle la composition phospholipidique comprend
phosphatidylcholine : 54 %,
sphingomyéline : 19 %,
phosphatidyléthanolamine : 8 %,
phosphatidylsérine : 4 %,
phosphatidylinositol : 3 %
et cholestérol : 10 %
dans laquelle les chiffres sont des pourcentages en poids.

3. Une vésicule phospholipidique telle que revendiquée dans n'importe lesquelles des revendications précédentes, dans laquelle la composition phospholipidique comporte de la lysolécithine.

4. Une vésicule phospholipidique telle que revendiquée dans la revendication 3, dans laquelle la composition phospholipidique comporte de 0 % à 3 % en poids de lysolécithine.

5. Une vésicule phospholipidique telle que revendiquée dans la revendication 3 ou la revendication 4, dans laquelle la composition phospholipidique de la vésicule comporte 2 % de lysolécithine.

6. Une vésicule phospholipidique telle que revendiquée dans la revendication 3 ou la revendication 4, dans laquelle la vésicule est un corps lamellaire isolé du corps mammalien.

7. Une vésicule phospholipidique telle que revendiquée dans n'importe laquelle des revendications précédentes, destinée à être utilisée comme agent de vaccination.

8. Une vésicule phospholipidique telle que revendiquée dans les revendications 1 à 7, dans laquelle se trouve une protéine possédant une séquence peptidique qui est homologue ou identique à celle énoncée dans la séquence ID No 1 ou No 2, et possédant une séquence peptidique supplémentaire qui est homologue ou identique à celles énoncées dans la séquence ID No 3 ou No 4, dans laquelle la protéine résultante est capable de faire office d'adjuvant pour produire une réponse immunitaire.

9. Une vésicule phospholipidique telle que revendiquée dans la revendication 8, dans laquelle la séquence peptidique qui est homologue montre entre 99 % et 75 % d'homologie à SEQ ID No 1, SEQ ID No 2, SEQ ID No 3 ou SEQ ID No 4 respectivement.

10. Une vésicule phospholipidique telle que revendiquée dans la revendication 8, dans laquelle la séquence peptidique qui est homologue montre 99% d'homologie à SEQ ID No 1, SEQ ID No 2, SEQ ID No 3 ou SEQ ID No 4 respectivement.

11. Une vésicule phospholipidique selon la revendication 8 dans laquelle se trouve une protéine possédant une séquence peptidique telle qu'énoncée dans la séquence ID No 1 ou ID No 2, et possédant en outre une séquence peptidique telle qu'énoncée dans la séquence ID No 3 ou ID No 4.

12. Une vésicule phospholipidique telle que revendiquée dans n'importe laquelle des revendications 1 à 7, dans laquelle se trouve du dimycolate de tréhalose.

13. Une vésicule phospholipidique telle que revendiquée dans n'importe laquelle des revendications 1 à 7 incorporant la séquence peptidique de SP-A 77 à 110 pour la présentation d'antigène à des cellules présentatrices d'antigènes autologues professionnelles et non professionnelles in vitro pour susciter une réponse immunothérapeutique chez un hôte.

14. Une vésicule phospholipidique telle que revendiquée dans n'importe laquelle des revendications 1 à 7 incorporant du dimycolate de tréhalose et la séquence peptidique de SP-A 77 à 110.

15. Cellules présentatrices d'antigènes modifiées pour induire une réponse immunitaire cellulaire comprenant des cellules présentatrices d'antigènes isolées d'un corps mammalien, modifiées par la reprise de vésicules phospholipidiques selon n'importe laquelle des revendications 1 à 12.

16. Cellules présentatrices d'antigènes modifiées telles que revendiquées dans la revendication 15, dans lesquelles les cellules présentatrices d'antigènes sont des cellules dendritiques.

17. Une méthode pour incorporer des antigènes dans des cellules dendritiques, comprenant l'étape de mélanger des vésicules phospholipidiques selon les revendications 1 à 7 avec des cellules dendritiques.

18. Une composition pharmaceutique pour induire une réponse immunitaire comprenant une vésicule phospholipidique selon les revendications 1 à 7, ou des cellules présentatrices d'antigènes modifiées selon les revendications 15 et 16, et un support acceptable d'un point de vue pharmaceutique.
